# EUROPEAN PATENT APPLICATION

(11) **EP 3 919 610 A1**
(43) Date of publication of application: **08.12.2021**
(21) Application number: 21175642.4
(22) Date of filing: 25.05.2021
(51) Int. Cl.: C12M 3/06, C12M 1/12, C12M 3/00, C12M 1/42

(54) **METHOD OF PRODUCING CELL-CONTAINING CONTAINER**

(30) Priority: 27.05.2020 JP 2020092200
(71) Applicant: Ricoh Company, Ltd., Tokyo 143-8555 (JP)
(72) Inventor: NAKAYAMA, Tomoaki, Tokyo 143-8555 (JP); IJICHI, Rie, Tokyo 143-8555 (JP); ARATANI, Tomoyuki, Tokyo 143-8555 (JP); KOSHIZUKA, Shinnosuke, Tokyo 143-8555 (JP)
(74) Representative: Fairbairn, Angus Chisholm

(57) **Abstract**

A method of producing a cell-containing container is provided, including a process in which pluripotent stem cells that have been induced to differentiate into neurons, and astrocytes are mixed and seeded in a cell culture container in which an electrode array is arranged on a culture surface, and a process in which the cell culture container is incubated, and as a result, the pluripotent stem cells and the astrocytes adhere to the culture surface, and the pluripotent stem cells differentiate into neurons.

## Description

### BACKGROUND OF THE INVENTION

### Field of the Invention

The present invention relates to a method of producing a cell-containing container. More specifically, the present invention relates to a method of producing a cell-containing container, a method of inhibiting separation of neurons from a culture surface when the neurons are arranged in a cell culture container in which an electrode array is arranged on the culture surface, and a cell-containing container. Priority is claimed on Japanese Patent Application No. 2020-092200, filed May 27, 2020, the content of which is incorporated herein by reference.

### Description of Related Art

An action potential of neurons may be used to evaluate a drug efficacy or toxicity against neurons. As one method of detecting and evaluating an action potential of neurons, an evaluation method using a microelectrode array (MEA) is known. The MEA is an array of microelectrodes arranged on a substrate for cell culture and can detect electrical activity of cells.

Incidentally, it is known that, when neurons derived from human induced pluripotent stem cells (iPS) cells are cultured on the MEA and the action potential is detected, it is necessary to culture the neurons at a higher density and for a longer time than when non-human animal-derived neurons are used.

In addition, when iPS cell-derived cells are handled in their own laboratory, know-how and high costs are required for cell maintenance and differentiation, and thus there is a demand for ready-to-use plates that are purchased as assay plates on which cells are seeded.

For example, Patent Document 1 (Japanese Unexamined Patent Application, First Publication No. 2017-528127) describes that excitatory neurons, inhibitory neurons and astrocytes, all of which were differentiated, were mixed and cultured on MEA plates.

### SUMMARY OF THE INVENTION

The inventors found that, when iPS-derived neurons are cultured at a high density for a long time, the cells may aggregate and separate from a culture surface of a culture container. In particular, during transport of ready-to-use plates, cells are subjected to vibration and are thus likely to separate. An object of the present invention is to provide a technology for inhibiting separation of neurons from a culture surface of a culture container.

A method of producing a cell-containing container according to the present invention includes a process in which pluripotent stem cells that have been induced to differentiate into neurons, and astrocytes are mixed and seeded in a cell culture container in which an electrode array is arranged on a culture surface, and a process in which the cell culture container is incubated, and as a result, the pluripotent stem cells and the astrocytes adhere to the culture surface, and the pluripotent stem cells differentiate into neurons.

A method of inhibiting separation of neurons from a culture surface when the neurons are arranged in a cell culture container in which an electrode array is arranged on the culture surface according to the present invention includes a process in which pluripotent stem cells that have been induced to differentiate into neurons, and astrocytes are mixed and seeded in the cell culture container, and a process in which the cell culture container is incubated, and as a result, the pluripotent stem cells and the astrocytes adhere to the culture surface, and the pluripotent stem cells differentiate into neurons.

A cell-containing container according to the present invention includes neurons, astrocytes and a medium, in which the neurons and the astrocytes adhere to a culture surface of the cell-containing container, and an adhesion area of the neurons and the astrocytes to the culture surface is 0.5 mm² or more per 80,000 total cells including the neurons and the astrocytes.

According to the present invention, it is possible to provide a technology for inhibiting separation of neurons from a culture surface of a culture container.

### BRIEF DESCRIPTION OF THE DRAWINGS

FIG. 1(a) is a representative photomicroscopic image of neurons evaluated as having an aggregation level of 1 in Experiment Example 1.
FIG. 1(b) is a representative photomicroscopic image of neurons evaluated as having an aggregation level of 2 in Experiment Example 1.
FIG. 1(c) is a representative photomicroscopic image of neurons evaluated as having an aggregation level of 3 in Experiment Example 1.
FIG. 1(d) is a representative photomicroscopic image of neurons evaluated as having an aggregation level of 4 in Experiment Example 1.
FIG. 2(a) is a photomicroscopic image of neurons co-cultured with astrocytes in Experiment Example 2.
FIG. 2(b) is a photomicroscopic image of neurons not co-cultured with astrocytes in Experiment Example 2.
FIG. 3(a) is a graph showing the results obtained by measuring an action potential of neurons co-cultured with astrocytes in Experiment Example 2.
FIG. 3(b) is a graph showing the results obtained by measuring an action potential of neurons not co-cultured with astrocytes in Experiment Example 2.
FIG. 4 is a graph showing the change in aggregation level of neurons over time in Experiment Example 2.
FIGS. 5(a) to 5(f) are photomicroscopic images showing the observation results obtained by mixing pluripotent stem cells that have been induced to differentiate into neurons, and astrocytes at various ratios and culturing them, and graphs showing the measurement results of the action potential of neurons in Experiment Example 3.

### DETAILED DESCRIPTION OF THE INVENTION

### [Method of producing cell-containing container]

In one embodiment of the present invention, a method of producing a cell-containing container is provided, including a process in which pluripotent stem cells that have been induced to differentiate into neurons, and astrocytes are mixed and seeded in a cell culture container in which an electrode array is arranged on a culture surface, and a process in which the cell culture container is incubated, and as a result, the pluripotent stem cells and the astrocytes adhere to the culture surface, and the pluripotent stem cells differentiate into neurons.

As will be described below in examples, according to the production method of the present embodiment, when pluripotent stem cells that have been induced to differentiate into neurons, and astrocytes are mixed and seeded, it is possible to inhibit separation of the neurons from a culture surface of a cell culture container (MEA plate) in which an electrode array is arranged. The reason for this is not clear, but the inventors speculated that it is due to astrocytes having high adhesiveness to both the culture substrate and the neurons. In addition, when pluripotent stem cells before differentiation that have been induced to differentiate into neurons are seeded instead of differentiated neurons, it is possible to further inhibit separation of neurons.

When separation of neurons is inhibited, the action potential of the neurons can be detected well and evaluated using the MEA or the like. In addition, when aggregation of neurons is inhibited, the state of the neurons can be evaluated well by other analyzing methods such as immunostaining.

Examples of pluripotent stem cells include embryonic stem cells (ES cells) and induced pluripotent stem cells. Examples of induced pluripotent stem cells include nuclear transfer embryonic stem cells (ntES cells) and iPS cells. Among these, iPS cells are preferable as pluripotent stem cells.

iPS cells may be derived from healthy people or may be derived from patients having various nervous system diseases. In addition, the cells may be cells that have been subjected to various types of gene editing, and may be, for example, cells that have been engineered to have genes that are causes or risk factors of various nervous system diseases according to gene editing.

When iPS cells are cells derived from patients having various nervous system diseases, they can be used to construct a disease model of the nervous system. Nervous system diseases are not particularly limited, and examples thereof include neurodegenerative disease, autism, epilepsy, attention-deficit hyperactivity disorder (ADHD), schizophrenia, and bipolar disorder. Examples of neurodegenerative diseases include Alzheimer's disease, Parkinson's disease, and amyotrophic lateral sclerosis.

The animal species from which neurons are derived are not particularly limited, and examples thereof include humans, monkeys, dogs, cows, horses, sheep, pigs, rabbits, mice, rats, guinea pigs, and hamsters. Among these, humans are preferable.

In addition, neurons may be of a single type or a mixture of two or more types of neurons. Neurons can be roughly classified into, for example, peripheral neurons and central neurons. Examples of peripheral neurons include sensory neurons, motor neurons, and autonomic neurons. Examples of central neurons include intervening neurons and projection neurons. Examples of projection neurons include cortical neurons, hippocampal neurons, and amygdala neurons. In addition, central neurons can be roughly classified into excitatory neurons and inhibitory neurons. Glutamic acid-operated neurons mainly responsible for excitatory transmission in the central nervous system and γ-aminobutyric acid (GABA)-ergic neurons mainly responsible for inhibitory transmission may be exemplified.

Other neurons that release neuromodulators include cholinergic neurons, dopaminergic neurons, noradrenergic neurons, serotonergic neurons, histaminergic neurons and the like.

The pluripotent stem cells that have been induced to differentiate into neurons are pluripotent stem cells that are destined to differentiate into neurons. Specific examples of such cells include cells of Quick-Neuron (TM) series (commercially available from Elixirgen Scientific). These cells are pluripotent stem cells that have been induced to differentiate into neurons and differentiate into functionally mature neurons in about 10 days.

The astrocytes may be primary cultured cells or may be astrocytes that have been induced to differentiate from pluripotent stem cells. Examples of pluripotent stem cells include the same as those described above.

In the process in which pluripotent stem cells that have been induced to differentiate into neurons, and astrocytes are mixed and seeded in a cell culture container in which an electrode array is arranged on a culture surface, the ratio of the pluripotent stem cells and the astrocytes to be mixed (the number of pluripotent stem cells : the number of astrocytes) is preferably 1:1 to 4:1.

As will be described below in examples, when pluripotent stem cells and astrocytes are mixed at a ratio within the above range, the differentiated neurons maintain an aggregation level at which the action potential can be detected well.

In addition, a total cell number of the pluripotent stem cells and the astrocytes per unit area of the culture surface of the cell culture container is preferably 2,500 to 300,000 cells/cm², for example, 2,800 to 300,000 cells/cm².

A period for which pluripotent stem cells and astrocytes are mixed and seeded and the cell culture container is then incubated can be appropriately set depending on purposes, and is preferably a time sufficient for at least pluripotent stem cells to differentiate into neurons. For example, when cells of Quick-Neuron (TM) series (commercially available from Elixirgen Scientific) are used, the period is at least about 10 days after cell seeding.

For example, when the action potential is detected in neurons that have been induced to differentiate from human iPS cells, the period can be, for example, 30 days or longer, 40 days or longer, 50 days or longer, 60 days or longer, or 70 days or longer, from when pluripotent stem cells and astrocytes are mixed and seeded.

In addition, depending on purposes, mature neurons are preferable, and for example, neurons in which the expression of any of marker genes of Tubulin beta3, MAP2, NeuN, 160kDa Neurofilament, 200kDa Neurofilament, NSE, PSD93, and PSD95 is positive are preferable.

The cell culture container may be a container generally used for cell culture, and examples thereof include a dish and a well plate. The diameter of the dish, the number of wells per well plate, and the like can be appropriately selected depending on applications. An electrode array is arranged on the culture surface of the cell culture container. That is, the cell-containing container obtained by the production method of the present embodiment is a microelectrode array (MEA) plate. The number of electrodes of the MEA and the like can be appropriately selected depending on applications.

Examples of materials of the culture surface of the culture container include organic materials and inorganic materials described below. These may be used alone or two or more thereof may be used in combination.

The organic material is not particularly limited, and can be appropriately selected depending on purposes, and examples thereof include acrylic materials such as polyethylene terephthalate (PET), polystyrene (PS), polycarbonate (PC), triacetyl cellulose (TAC), polyimide (PI), nylon (Ny), low-density polyethylene (LDPE), medium-density polyethylene (MDPE), vinyl chloride, vinylidene chloride, polyphenylene sulfide, polyether sulfone, polyethylene naphthalate, polypropylene, and urethane acrylate, silicone materials such as cellulose and polydimethylsiloxane (PDMS), polyvinyl alcohol (PVA), alginate metal salts such as calcium alginate, and gel-like materials such as polyacrylamide, methylcellulose, and agarose.

The inorganic material is not particularly limited, and can be appropriately selected depending on purposes, and examples thereof include glass and ceramics.

The culture surface of the culture container may be coated with a coating agent. As the coating agent, those generally used for cell culture can be appropriately used, and examples thereof include collagen, Matrigel (registered trademark, commercially available from Corning Inc.), Geltrex (commercially available from Thermo Fisher Scientific), PLO (commercially available from Sigma-Aldrich Co. LLC), PDLO (commercially available from Sigma-Aldrich Co. LLC), fibronectin, fibrinogen, gelatin, polyethyleneimine (PEI), and laminin.

As the medium, a medium in which necessary components are added to a basal medium can be used. Examples of basal mediums include BrianPhys (commercially available from STEMCELL Technologies), Neurobasal (commercially available from Thermo Fisher Scientific), Dulbecco's Modified Eagle's Medium (DMEM), Ham F12 medium (Ham's Nutrient Mixture F12), D-MEM/F12 medium, McCoy's 5A medium, Eagle's MEM medium (Eagle's Minimum Essential Medium, EMEM), α MEM medium (alpha Modified Eagle's Minimum Essential Medium, αMEM), MEM medium (Minimum Essential Medium), RPMI 1640 (Roswell Park Memorial Institute-1640) medium, Iscove's Modified Dulbecco's Medium (IMDM), MCDB131 medium, William's medium E, IPL41 medium, Fischer's medium, M199 medium, high performance improved 199 medium (High Performance Medium 199), StemPro34 (commercially available from Thermo Fisher Scientific), X-VIVO 10 (commercially available from Chembrex), X-VIVO 15 (commercially available from Chembrex), HPGM (commercially available from Chembrex), StemSpan H3000 (commercially available from STEMCELL Technologies), StemSpanSFEM (commercially available from STEMCELL Technologies), StemlineII (commercially available from Sigma-Aldrich Co. LLC), QBSF-60 (commercially available from Quality Biological), StemProhESCSFM (commercially available from Thermo Fisher Scientific), Essential 8 (registered trademark) medium (commercially available from Thermo Fisher Scientific), mTeSR1 or mTeSR2 medium (commercially available from STEMCELL Technologies), ReproFF or ReproFF2 (commercially available from Reprocell Inc.), PSGro hESC/iPSC medium (commercially available from System Biosciences), NutriStem (registered trademark) medium (commercially available from Biological Industries), CSTI-7 medium (commercially available from Cell Science & Technology Institute, Inc.), MesenPRO RS medium (commercially available from Thermo Fisher Scientific), MF-Medium (registered trademark) mesenchymal stem cell growth medium (commercially available from Toyobo Co., Ltd.), Sf-900II (commercially available from Thermo Fisher Scientific), and Opti-Pro (commercially available from Thermo Fisher Scientific). These may be used alone or two or more thereof may be used in combination.

In addition, examples of additives added to basal mediums include those generally used for culturing neurons, and examples thereof include SM1 supplement (commercially available from STEMCELL Technologies), N2 supplement A (commercially available from STEMCELL Technologies), rat astrocyte culture supernatant (commercially available from FUJIFILM Wako Pure Chemical Corporation), human astrocyte culture supernatant (commercially available from ScienCell Research Laboratories), Component N (commercially available from Elixirgen Scientific), Component G2 (commercially available from Elixirgen Scientific), Component P (commercially available from Elixirgen Scientific), N2 Supplement (commercially available from Thermo Fisher Scientific), iCell Neural Supplement B (commercially available from CDI), iCell Nervous System Supplement, and B-27 plus (commercially available from Thermo Fisher Scientific).

### [Method of inhibiting separation of neurons]

In one embodiment, the present invention provides a method of inhibiting separation of neurons from a culture surface when the neurons are arranged in a cell culture container in which an electrode array is arranged on the culture surface, the method including a process in which pluripotent stem cells that have been induced to differentiate into neurons, and astrocytes are mixed and seeded in the cell culture container, and a process in which the cell culture container is incubated, and as a result, the pluripotent stem cells and the astrocytes adhere to the culture surface, and the pluripotent stem cells differentiate into neurons.

According to the method of the present embodiment, when pluripotent stem cells that have been induced to differentiate into neurons, and astrocytes are mixed and seeded, it is possible to inhibit separation of the neurons from a culture surface of a cell culture container (MEA plate) in which an electrode array is arranged. In addition, when pluripotent stem cells before differentiation that have been induced to differentiate into neurons are seeded instead of differentiated neurons, it is possible to further inhibit separation of neurons.

In the present embodiment, in the pluripotent stem cells, the pluripotent stem cells that have been induced to differentiate into neurons, astrocytes, the cell culture container, a mixing ratio of the pluripotent stem cells and the astrocytes, the seeding process, the total cell number of the pluripotent stem cells and the astrocytes per unit area of the culture surface, the incubation period, and the like are the same as those described above.

### [Cell-containing container]

In one embodiment, the present invention provides a cell-containing container including neurons, astrocytes and a medium, and in which the neurons and the astrocytes adhere to a culture surface of the cell-containing container, and an adhesion area of the neurons and the astrocytes to the culture surface is 0.5 mm² or more per 80,000 total cells including the neurons and the astrocytes.

In the cell-containing container of the present embodiment, since neurons and astrocytes are co-cultured, separation of the neurons from the culture surface of the culture container is inhibited.

In the cell-containing container of the present embodiment, since aggregation and separation of neurons are inhibited, the action potential of the neurons can be detected well and evaluated using the MEA or the like. In addition, when aggregation of neurons is inhibited, the state of the neurons can be evaluated well by other analyzing methods such as immunostaining.

The cell-containing container of the present embodiment can be produced according to, for example, a method of producing a cell-containing container including a process in which pluripotent stem cells that have been induced to differentiate into neurons, and astrocytes are mixed and seeded in a cell culture container, and a process in which the cell culture container is incubated, and as a result, the pluripotent stem cells and the astrocytes adhere to the culture surface and the pluripotent stem cells differentiate into neurons. An electrode array may be arranged on the culture surface of the cell-containing container.

Here, when pluripotent stem cells that have been induced to differentiate into neurons, and astrocytes are mixed and seeded, it is possible to inhibit separation of the neurons from the culture surface of the cell culture container. In addition, when pluripotent stem cells before differentiation that have been induced to differentiate into neurons are seeded instead of differentiated neurons, it is possible to further inhibit separation of neurons.

The cell-containing container of the present embodiment may contain oligodendrocytes, microglia, and the like as well as neurons and astrocytes. The adhesion area of these cells to the culture surface of the culture container is 0.5 mm² or more, preferably 0.949 mm² or more, more preferably 3 mm² or more, and still more preferably 3.14 mm² or more per 80,000 total cells. In addition, the upper limit of the adhesion area of cells to the culture surface of the culture container is preferably about 28.2 mm².

In this specification, the adhesion area of the cells to the culture surface of the culture container is an area on the culture surface of a region in which an aggregation of regions (cell bodies) in which nuclei are present adhere to the culture surface among cells including neurons and astrocytes. That is, the area of the region in which only protrusions (dendrites and axons) of neurons and protrusions of astrocytes adhere to the culture surface of the culture container is not included in the above adhesion area.

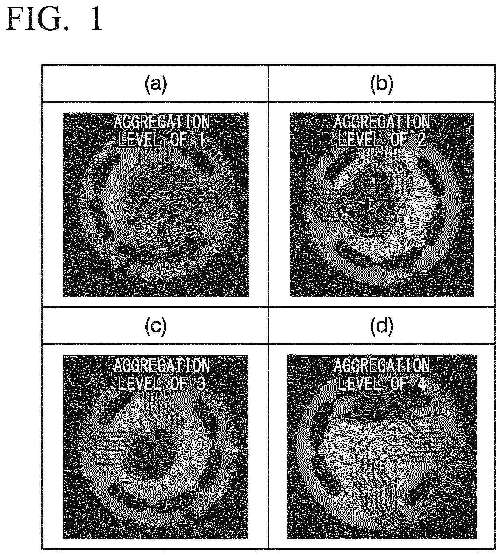

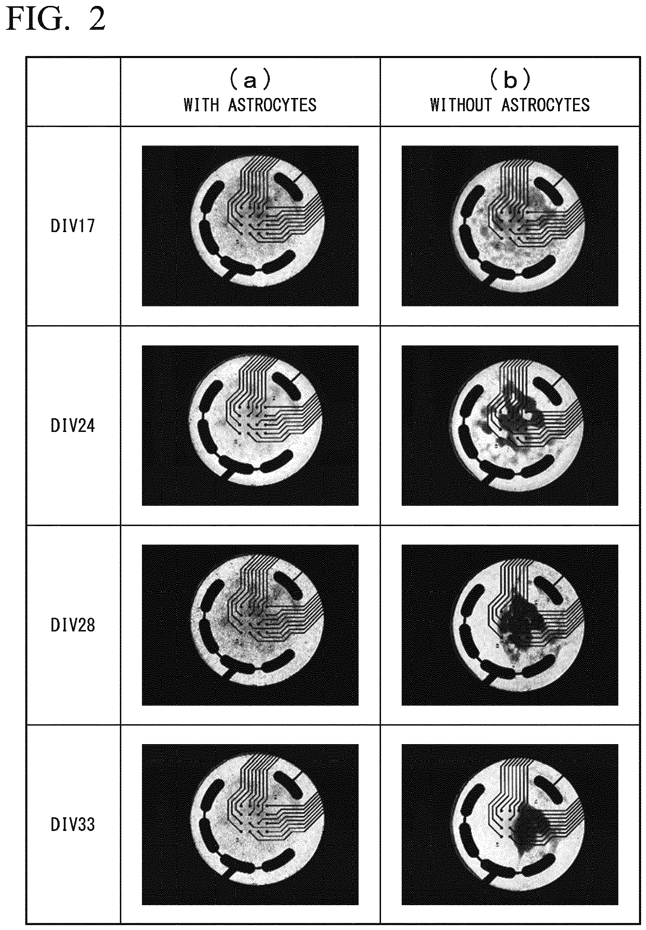

The aggregate of cell bodies can be clearly distinguished from protrusions according to observation under an optical microscope or the like. The aggregate of cell bodies is observed as a dark color region of the cell mass, for example, as shown in FIGS. 1(a) to 1(d), and FIGS. 2(a) and 2(b) to be described below in examples.

Alternatively, a region in which nuclei are present may be identified by staining cells with a nucleus-specific dyeing agent and observing fluorescence. Examples of nucleus-specific dyeing agents include Hoechst 33342 and 4',6-diamidino-2-phenylindole (DAPI).

In the cell-containing container of the present embodiment, depending on purposes, mature neurons are preferable, and for example, neurons in which expression of any of marker genes of Tubulin beta3, MAP2, NeuN, 160kDa Neurofilament, 200kDa Neurofilament, NSE, PSD93, and PSD95 is positive are preferable.

In the present embodiment, in the pluripotent stem cells, the pluripotent stem cells that have been induced to differentiate into neurons, astrocytes, the cell culture container, a mixing ratio of the pluripotent stem cells and the astrocytes, the seeding process, the total cell number of the pluripotent stem cells and the astrocytes per unit area of the culture surface, the incubation period, and the like are the same as those described above.

### Examples

Next, the present invention will be described in more detail with reference to examples, but the present invention is not limited to the following examples.

### [Experiment Example 1]

### (Examination of aggregation level of neurons and whether action potential could be detected)

The inventors previously revealed that, when human neurons were cultured with different concentrations of glucose in the medium, the aggregation level of the neurons differed. In this experiment example, human neurons were cultured on the MEA plate while adjusting the concentration of glucose by changing the amount of medium replaced to prepare neurons with different aggregation levels. In addition, the aggregation level of the neurons and whether the action potential could be detected were examined.

As human neurons, Human iPSC-derived GABAergic Neurons (commercially available from Elixirgen Scientific) were used.

In addition, as the MEA plate, an MEA plate (model number "M768-tMEA-48W," commercially available from Axion Biosystems) having a microelectrode array was used. The culture surface of the MEA plate that was used was coated with polyethyleneimine (PEI, commercially available from Thermo Fisher Scientific) and laminin (commercially available from Thermo Fisher Scientific).

From the day of cell seeding (0 days in vitro, hereinafter sometimes referred to as "DIV0") to the 5^{th} day (hereinafter sometimes referred to as "DIV5," the same applies hereinafter), "Quick-Neuron (TM) GABAergic Maintenance Medium" (product name, commercially available from Elixirgen Scientific) was used as the medium. The concentration of glucose in the medium was 5 mmol/L. After DIV6, "Complete Brainpys Medium" (product name, commercially available from CDI) was used as the medium. The concentration of glucose in the medium was 0.5 mmol/mL.

Subsequently, the cells cultured for 42 days after seeding were observed under a microscope, and an aggregation level thereof was evaluated. The evaluation criteria for the aggregation level are as follows. It was found that, as the aggregation level was higher, the adhesion area of the cells to the culture surface was smaller, and the cells were likely to be separated from the culture surface.

### <<Evaluation criteria for aggregation level>>

1: The adhesion area of the cells to the culture surface was 3.14 mm² or more and 28.2 mm² or less per 80,000 cells
2: The adhesion area of the cells to the culture surface was 0.949 mm² or more and less than 3.14 mm² per 80,000 cells
3: The adhesion area of the cells to the culture surface was 0.196 mm² or more and less than 0.949 mm² per 80,000 cells
4: The adhesion area of the cells to the culture surface was 0 mm² or more and less than 0.196 mm² per 80,000 cell

FIG. 1(a) is a representative photomicroscopic image of neurons evaluated as having an aggregation level of 1. FIG. 1(b) is a representative photomicroscopic image of neurons evaluated as having an aggregation level of 2. FIG. 1(c) is a representative photomicroscopic image of neurons evaluated as having an aggregation level of 3. FIG. 1(d) is a representative photomicroscopic image of neurons evaluated as having an aggregation level of 4.

Subsequently, the action potential of the neurons was measured and evaluated using a microelectrode array. The evaluation criteria for the action potential are as follows, and it was determined whether the action potential could be detected. In addition, the following Table 1 shows the evaluation results of the aggregation level and the action potential.

### <<Evaluation criteria for action potential>>

A: The action potential could be detected well.
B: The action potential could be detected.
C: The action potential could not be detected.

**[Table 1]**

| Aggregation level | 1 | 2 | 3 | 4 |
|---|---|---|---|---|
| Determination of whether action potential could be detected | A | B | C | C |

As a result, it was revealed that, when neurons evaluated as having an aggregation level of 1 were used, the action potential could be detected well. In addition, it was revealed that, when neurons evaluated as having an aggregation level of 3 or higher were separated from the MEA, the action potential of the neurons could not be detected.

### [Experiment Example 2]

### (Co-culture of neurons and astrocytes)

Pluripotent stem cells that had been induced to differentiate into neurons, and astrocytes were mixed, cultured on the MEA plate, and observed.

As the pluripotent stem cells that had been induced to differentiate into neurons, Human iPSC-derived Mixed Neurons (commercially available from Elixirgen Scientific) were used. These cells were pluripotent stem cells that had been induced to differentiate into neurons and differentiated into functionally mature neurons in about 10 days. In addition, as the astrocytes, Human Primary Astrocytes (commercially available from Thermo Fisher Scientific) were used.

In addition, as the MEA plate, an MEA plate (model number "M768-tMEA-48W," commercially available from Axion Biosystems) having a microelectrode array was used. The culture surface of the MEA plate that was used was coated with polyethyleneimine (PEI, commercially available from Thermo Fisher Scientific) and laminin (commercially available from Thermo Fisher Scientific).

For each MEA plate, 1.2×10⁵ pluripotent stem cells that had been induced to differentiate into neurons and 3.0×10⁴ astrocytes were mixed and seeded. In addition, for comparison, MEA plates on which pluripotent stem cells that had been induced to differentiate into neurons, and astrocytes were seeded without mixing were prepared.

From the day of cell seeding (DIV0) to the 2^{nd} day (DIV2), "Quick-Neuron (TM) Mixed Maintenance Medium" (product name, commercially available from Elixirgen Scientific) was used as the medium. After DIV2, "Complete Neurobasal plus Medium" (product name, commercially available from Thermo Fisher Scientific) was used as the medium.

FIGS. 2(a) and 2(b) are photomicroscopic images showing the observation results of the cells of respective groups over time. FIG. 2(a) shows the results of neurons co-cultured with astrocytes, and FIG. 2(b) shows the results of neurons not co-cultured with astrocytes.

As a result, it was revealed that, when the pluripotent stem cells that had been induced to differentiate into neurons and the astrocytes were co-cultured, the differentiated neurons maintained the same shape as immediately after seeding even on the 33^{rd} day after seeding. On the other hand, it was revealed that neurons not co-cultured with astrocytes were aggregated over time and separated.

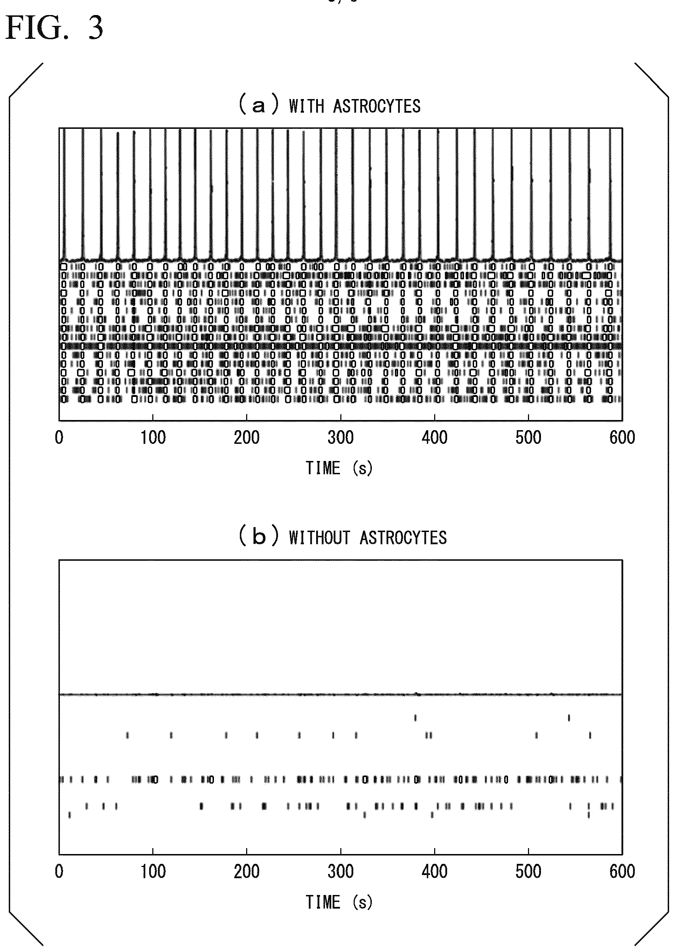

Subsequently, the action potential of the neurons was measured using an electrode array on the MEA plate on the 33^{rd} day after seeding. FIGS. 3(a) and 3(b) are graphs (raster plots) showing the measurement results of the action potential. FIG. 3(a) shows the results of neurons co-cultured with astrocytes, and FIG. 3(b) shows the results of neurons not co-cultured with astrocytes. As a result, it was revealed that the action potential could be detected well in the neurons co-cultured with astrocytes.

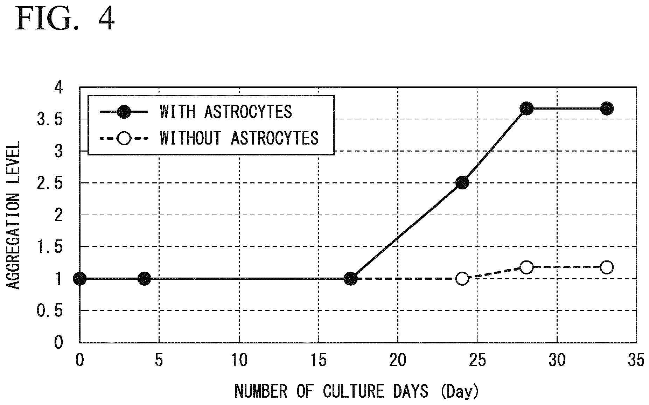

FIG. 4 is a graph showing the results of evaluating the aggregation levels of cells of respective groups over time. The evaluation criteria for the aggregation level were the same as in Experiment Example 1. As a result, it was revealed that the neurons co-cultured with astrocytes maintained the aggregation level at which the action potential could be detected. On the other hand, it was revealed that the aggregation level of the neurons not co-cultured with astrocytes increased over time.

### [Experiment Example 3]

### (Examination of mixing ratio of neurons and astrocytes)

Pluripotent stem cells that had been induced to differentiate into neurons, and astrocytes were mixed at various ratios, cultured on the MEA plate, and observed.

As the pluripotent stem cells that had been induced to differentiate into neurons, Human iPSC-derived GABAergic Neurons (commercially available from Elixirgen Scientific) were used. These cells were pluripotent stem cells that had been induced to differentiate into neurons and differentiated into functionally mature neurons in about a week. In addition, as the astrocytes, Human Primary Astrocytes (commercially available from Thermo Fisher Scientific) were used.

In addition, as the MEA plate, an MEA plate (model number "M768-tMEA-48W," commercially available from Axion Biosystems) having a microelectrode array was used. The culture surface of the MEA plate that was used was coated with polyethyleneimine (PEI, commercially available from Thermo Fisher Scientific) and laminin (commercially available from Thermo Fisher Scientific).

For each MEA plate, pluripotent stem cells that had been induced to differentiate into neurons, and astrocytes were mixed and seeded in the numbers of cells shown in the following Table 2.

**[Table 2]**

| Experiment Example | Pluripotent stem cells | Astrocytes | Ratio of numbers of cells (pluripotent stem cells: astrocytes) |
|---|---|---|---|
| 1 | 8.0×10⁴ | 2.0×10⁴ | 4:1 |
| 2 | 6.6×10⁴ | 3.3×10⁴ | 2:1 |
| 3 | 5.0×10⁴ | 5.0×10⁴ | 1:1 |
| 4 | 6.4×10⁴ | 1.6×10⁴ | 4:1 |
| 5 | 5.2×10⁴ | 2.6×10⁴ | 2:1 |
| 6 | 4.0×10⁴ | 4.0×10⁴ | 1:1 |

From the day of cell seeding (DIV0) to the 2^{nd} day (DIV2), "Quick-Neuron (TM) GABAergic Maintenance Medium" (product name, commercially available from Elixirgen Scientific) was used as the medium. After DIV2, "Complete Neurobasal plus Medium" (product name, commercially available from Thermo Fisher Scientific) was used as the medium. On the 28^{th} day after cell seeding, the action potential of the neurons was measured using an electrode array on the MEA plate for respective neurons.

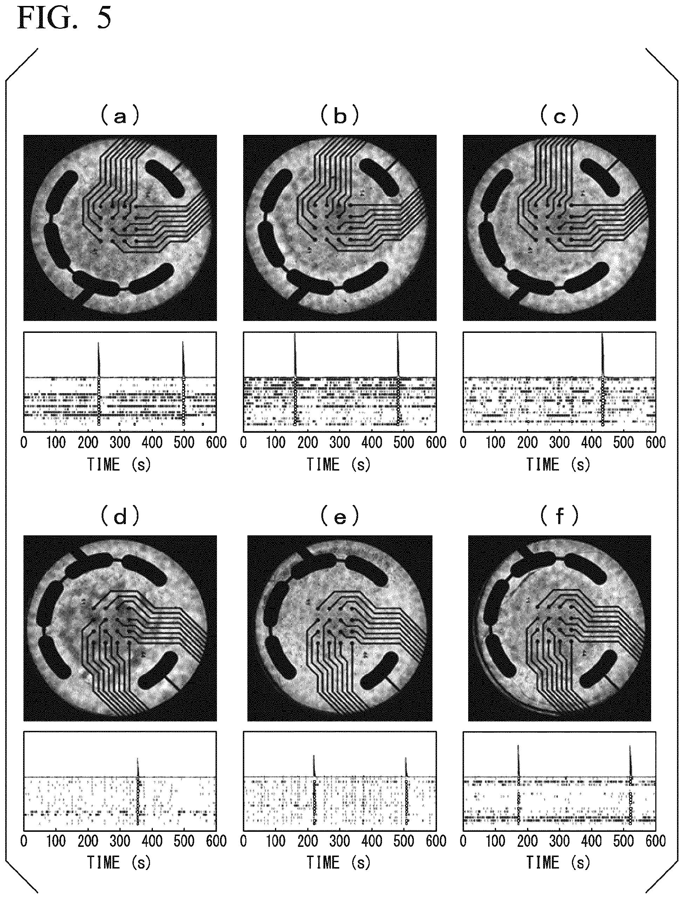

FIGS. 5(a) to 5(f) are photomicroscopic images showing the observation results of the neurons of respective groups and graphs (raster plots) showing the measurement results of the action potential of the neurons. FIG. 5(a) shows the results of Experiment No. 1 in Table 2, FIG. 5(b) shows the results of Experiment No. 2, FIG. 5(c) shows the results of Experiment No. 3, FIG. 5(d) shows the results of Experiment No. 4, FIG. 5(e) shows the results of Experiment No. 5, and FIG. 5(f) shows the results of Experiment No. 6.

As a result, it was revealed that, at any of the mixing ratios, the neurons co-cultured with astrocytes maintained the aggregation level at which the action potential could be detected well.

The present invention includes the following aspects.
[1] A method of producing a cell-containing container, including a process in which pluripotent stem cells that have been induced to differentiate into neurons, and astrocytes are mixed and seeded in a cell culture container in which an electrode array is arranged on a culture surface, and a process in which the cell culture container is incubated, and as a result, the pluripotent stem cells and the astrocytes adhere to the culture surface, and the pluripotent stem cells differentiate into neurons.
[2] The production method according to [1], wherein, in the seeding process, the ratio of the pluripotent stem cells and the astrocytes to be mixed (the number of pluripotent stem cells : the number of astrocytes) is 1:1 to 4:1.
[3] The production method according to [1] or [2], wherein, in the seeding process, a total cell number of the pluripotent stem cells and the astrocytes per unit area of the culture surface is 2,500 to 300,000 cells/cm².
[4] A method of inhibiting separation of neurons from a culture surface when the neurons are arranged in a cell culture container in which an electrode array is arranged on the culture surface, the method including: a process in which pluripotent stem cells that have been induced to differentiate into neurons, and astrocytes are mixed and seeded in the cell culture container; and a process in which the cell culture container is incubated, and as a result, the pluripotent stem cells and the astrocytes adhere to the culture surface, and the pluripotent stem cells differentiate into neurons.
[5] A cell-containing container, including: neurons, astrocytes and a medium, wherein the neurons and the astrocytes adhere to a culture surface of the cell-containing container, and wherein an adhesion area of the neurons and the astrocytes to the culture surface is 0.5 mm² or more per 80,000 total cells including the neurons and the astrocytes.
[6] The cell-containing container according to [5], wherein an electrode array is arranged on the culture surface.

### [Patent Document]

[Patent Document 1] Published Japanese Translation No. 2017-528127 of the PCT International Publication

While preferred embodiments of the invention have been described and illustrated above, it should be understood that these are exemplary of the invention and are not to be considered as limiting. Additions, omissions, substitutions, and other modifications can be made without departing from the spirit or scope of the present invention. Accordingly, the invention is not to be considered as being limited by the foregoing description, and is only limited by the scope of the appended claims.

## Claims

1. A production method of producing a cell-containing container, comprising:
a process in which pluripotent stem cells that have been induced to differentiate into neurons, and astrocytes are mixed and seeded in a cell culture container in which an electrode array is arranged on a culture surface; and
a process in which the cell culture container is incubated, and as a result, the pluripotent stem cells and the astrocytes adhere to the culture surface, and the pluripotent stem cells differentiate into neurons.

2. The production method according to Claim 1, wherein, in the seeding process, the ratio of the pluripotent stem cells and the astrocytes to be mixed (the number of pluripotent stem cells : the number of astrocytes) is 1:1 to 4:1.

3. The production method according to Claim 1 or 2, wherein, in the seeding process, a total cell number of the pluripotent stem cells and the astrocytes per unit area of the culture surface is 2,500 to 300,000 cells/cm².

4. A method of inhibiting separation of neurons from a culture surface when the neurons are arranged in a cell culture container in which an electrode array is arranged on the culture surface, the method comprising:
a process in which pluripotent stem cells that have been induced to differentiate into neurons, and astrocytes are mixed and seeded in the cell culture container; and
a process in which the cell culture container is incubated, and as a result, the pluripotent stem cells and the astrocytes adhere to the culture surface, and the pluripotent stem cells differentiate into neurons.

5. A cell-containing container, comprising:
neurons, astrocytes and a medium,
wherein the neurons and the astrocytes adhere to a culture surface of the cell-containing container, and
wherein an adhesion area of the neurons and the astrocytes to the culture surface is 0.5 mm² or more per 80,000 total cells including the neurons and the astrocytes.

6. The cell-containing container according to Claim 5, wherein an electrode array is arranged on the culture surface.
